# EUROPEAN PATENT SPECIFICATION

(11) **EP 4 389 090 B1**
(45) Date of publication and mention of the grant of the patent: **21.10.2026**
(21) Application number: 23216897.1
(22) Date of filing: 14.12.2023
(51) Int. Cl.: A61F 13/15

(54) **APPARATUS FOR DISTRIBUTING ABSORBENT FIBERS ONTO A SUBSTRATE**
VORRICHTUNG ZUM VERTEILEN VON ABSORBIERENDEN FASERN AUF EIN SUBSTRAT
APPAREIL POUR DISTRIBUER DES FIBRES ABSORBANTES SUR UN SUBSTRAT

(30) Priority: 19.12.2022 EP 22214725
(43) Date of publication of application: 26.06.2024
(73) Proprietor: Ontex BV, 9255 Buggenhout (BE); Ontex Group NV, 9320 Erembodegem (BE)
(72) Inventor: Heege, Thomas, 56761 Düngenheim (DE)
(74) Representative: Saurat, Thibault

(56) References cited:
- US-A- 4 592 708
- US-A1- 2003 116 888
- US-A1- 2008 113 082

## Description

### TECHNICAL FIELD

The invention pertains to the technical field of absorbent articles and the method and apparatus to form the absorbent core of said absorbent article such as disposable diapers and the like. In particular, the invention concerns an apparatus suitable for distributing absorbent fibers onto a substrate and as well as an apparatus suitable for forming moulded absorbent material deposit structures to be used as absorbent cores for absorbent articles comprising said apparatus suitable for distributing absorbent fibers onto a substrate. The present disclosure also relates to a method for forming moulded absorbent material deposit structures to be used as absorbent cores using said apparatus.

### BACKGROUND

As known, disposable absorbent articles such as diapers or training pants for babies, sanitary napkins or towels or products for adult incontinence, comprises an absorbent core arranged in between a liquid permeable topsheet and a liquid impermeable backsheet with eventually an acquisition distribution layer (ADL) in between the topsheet and the absorbent core. The general purpose of such absorbent articles is to absorb, distribute and store various types of body exudates, while providing a high level of comfort and sense of dryness to the wearer during use of the absorbent article. In order to do so, the disposables typically comprise an absorbent core, or absorbent body, which is capable of quickly absorbing and retaining the exudates. The absorbent core comprises absorbent material such as cellulosic fibres or fluff or fluff pulp and/or of particles of superabsorbent material (SAP). The absorbent material is usually contained, encapsulated or enclosed in a core wrap. The core wrap can comprise one core wrap sheet extending above and under and on the side of the absorbent material thereby fully or at least partially covering the absorbent material. Alternatively, the core wrap can comprise two core wrap sheet, an upper core wrap and a lower absorbent core wrap, the absorbent material between arranged in between. In recent years, more and more absorbent cores comprise at least one channel substantially free of absorbent material. The at least one channel is formed by adhering the lower core wrap sheet to the upper core wrap sheet, or the portion of the sheet above to the portion of the sheet under when the core wrap comprises one single sheet.

The process and apparatus commonly used for the formation of absorbent bodies is generally described below.

The absorbent cores are manufactured using an apparatus comprising a defibrating device, such as a mill or more specifically a hammermill, which defibers, or pulverizes, a sheet of pulp material, resulting in pulp fibers. Additionally, the apparatus comprises a tube feeding superabsorbent particles within said duct and superabsorbent particles are discharged into said duct. In this way, the superabsorbent particles and the pulp fibers flow together within the duct. An airflow conveys the pulp fibers and superabsorbent particles to a forming pocket where the absorbent fibers and superabsorbent particles are deposited thereby forming a moulded absorbent material deposit structure. These process can be costly.

Documents US 2003/116888 A1, US 4 592 708 A or US 2008113082 A1 describe apparatuses for forming absorbent cores with costly processes.

It is thus an object of the present invention to provide a method and apparatus to make absorbent cores with a quantity of recycled fluff and/or freshly defibrated fluff as desired in order to balance absorbency (freshly defibrated fluff) and cost (recycled fluff).

### SUMMARY

The present invention pertains to an apparatus for distributing absorbent fibers onto a substrate, the apparatus comprising a defibrating station comprising :
a. a defibrating machine suitable for defibrating a sheet of pulp material and generating fresh absorbent fibers and a first airflow, and
b. a recycling duct by-passing the defibrating machine and conveying recycled absorbent fibers from a filtering apparatus,
wherein the defibrating machine and the recycling duct are fluidically connected to at least two ducts, the recycling duct comprises at least one flap to convey the recycled absorbent fibers into one, two or more of the ducts.

Such recycling duct enable to control the quantity of recycled fluff and/or freshly defibrated fluff in an absorbent core. The filtering apparatus is in fluidic connection with the recycling duct.

According to an embodiment, the apparatus comprises two ducts and the recycling duct comprises two flaps to convey the recycled absorbent fibers into one or two ducts.

According to an embodiment, said at least one flap is selected from a door flap, a butterfly flap, a drum flap, a sliding door flap.

According to an embodiment, the defibrating machine is a hammermill arranged in a drum housing, the recycling duct following the curvature of said drum housing.

The present disclosure also pertains to an an apparatus suitable for forming moulded absorbent material deposit structures to be used as absorbent cores for absorbent articles, the apparatus comprising:
- a forming unit comprising: a forming drum comprising an outer circumferential surface, a plurality of forming pockets arranged on the outer circumferential surface of said forming drum, each forming pocket comprising a mould cavity; and
- a feeding unit comprising:
   ∘ an apparatus for distributing fresh and/or recycled absorbent fibers onto a substrate as described above,
   ∘ at least one discharging conduits defined by a housing with a internal wall fluidically connecting the outlets of the defibrating station and the forming unit through which the fresh and/or recycled absorbent fibers and a first airflow flow.

According to an embodiment, the apparatus further comprises an apparatus for distributing superabsorbent particles comprising a discharge pipe for conveying a stream comprising superabsorbent particles and a second airflow, said discharge pipe comprising a conveying section and an outlet nozzle , said outlet nozzle comprising a straight portion and a deflecting portion wherein the deflecting portion comprises deflecting means arranged in a way that only a portion of the stream of superabsorbent particle and second airflow is deflected and the other portion of said stream is not deflected, wherein the discharge pipe extends partially within the discharging conduit, the outlet nozzle being arranged within the discharging conduit in a way that the discharge pipe sprays superabsorbent particles into the discharging conduit and into the mould cavity of the forming pockets.

According to an embodiment, the apparatus comprises two discharging conduit each conduit being fluidically connected to the defibrating station and each conduit comprising a discharge pipe extending partially within its respective discharging conduit, the outlet nozzle of each pipe being arranged within its respective discharging conduit.

Having two discharging conduits enables to make absorbent cores with 2 layers of absorbent material. It is possible with the apparatus described herein to control the quantity of recycled fluff and/or freshly defibrated fluff in each layer of absorbent core. The discharging conduits are fluidically connected to the ducts

According to an embodiment, the deflecting means define a first angle between the conveying section and the deflection portion, said first angle being comprised between 91° and 179°, preferably between 120° and 175°, more preferably between 150° and 170°.

According to an embodiment, the discharge pipe extends longitudinally, transversally and vertically, said outlet nozzle comprises a top wall, a bottom wall, a first and second side wall, each wall comprising a proximal end connected to the conveying section and a distal end arranged at the outlet of the discharging pipe, the distal end of the top wall extending longitudinally beyond the distal end of the bottom wall and/or the distal end of the first side wall extending longitudinally beyond the distal end of the second side wall.

According to an embodiment, the discharge pipe further comprises a first feeding tube conveying a first airflow, a second feeding tube conveying superabsorbent particles and a venturi section arranged at the junction between the first and second feeding tubes and the conveying section, the first and second feeding tubes and the conveying section all being fluidically connected, first and second feeding tubes being arranged upstream of the conveying section with respect to the direction of flow.

According to an embodiment, the outlet nozzle comprises a rectangular transversal cross section and the cross-sectional area of outlet nozzle is lesser than the cross-sectional area of the conveying section.

The present disclosure also relates to a method for forming moulded absorbent material deposit structures to be used as absorbent cores for absorbent articles using the apparatus described hereabove, the method comprising:
a) a defibrating step wherein a continuous sheet of absorbent material is defibrated by a defibrating machine at a defibrating station, thereby obtaining a plurality of fresh absorbent fibers;
b) a conveying step where a stream of recycled absorbent fibers is transported in the recycling duct;
c) a transport step wherein fresh and/or recycled absorbent fibers are conveyed by a first airflow flowing within the discharging conduit or ducts from the defibrating station and recycling duct to the forming pocket;
d) a feeding step wherein a specific amount of superabsorbent particles in conjunction with the second airflow is sprayed from the discharge pipe, into the discharging conduit via the outlet nozzle;
e) an accumulation step wherein the fresh and/or recycled absorbent fibers and the superabsorbent particles accumulate within the mould cavity of the forming pocket thereby forming moulded absorbent material deposit structures to be used as absorbent cores for absorbent articles.

### BRIEF DESCRIPTION OF THE DRAWINGS

The drawings and figures are illustrative in nature and not intended to limit the subject matter defined by the claims. The following detailed description can be understood when read in conjunction with the following drawings, where similar or identical structures are indicated with the same reference numerals in which:
- FIG. 1 illustrates a schematic side-view, or longitudinal cross-section, of an apparatus suitable for forming moulded absorbent material deposit structures to be used as absorbent cores for absorbent articles;
- FIG. 2 shows a schematic side-view, or longitudinal cross-section, of the defibrating station;
- FIG. 3 depicts a schematic top view and cross section of the recycling duct ;
- FIG. 4 represents a schematic top views and cross section of the outlet section of the recycling duct;
- FIG. 5 illustrates a view in perspective of the filtering apparatus;
- FIG. 6 shows a schematic cross section of an absorbent article;
- FIG. 7 illustrates a schematic side-view, or longitudinal cross-section, of a portion of the apparatus of FIG. 1, namely a schematic side-view of the portions of the discharging pipe feeding the superabsorbent particles and the duct conveying the absorbent fibers.

### DETAILED DESCRIPTION

As discussed hereabove, there remains a need for a more efficient way of producing absorbent cores balancing absorbency and cost. The present disclosure relates to an apparatus suitable for forming and distributing absorbent fibers onto a substrate and as well as an apparatus suitable for forming moulded absorbent material deposit structures to be used as absorbent cores for absorbent articles comprising said an apparatus suitable for distributing superabsorbent particles homogeneously onto a substrate. The present disclosure also relates to a method for forming moulded absorbent material deposit structures to be used as absorbent cores using said apparatus. However, other mixtures of materials may be produced employing the present invention, depending on the particular parameters desired in the absorbent body. Such alternative configurations and uses are contemplated as being within the scope of the present invention.

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-20% or less, preferably +/-((10))% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

The term "channel(s)" as used herein means fluid distribution means within the absorbent core adapted to favor exudate flow there along and are typically intended to exclude embossing patterns or ducts formed by compression from the meaning thereof and rather include structures that are substantially free of absorbent material instead of comprising compacted absorbent material. Channels are commonly known in the art. Channels herein are formed by joining upper and lower layers of a core wrap as will be described in more detail hereinbelow. Channels preferably comprise recessed regions forming visible grooves or passages typically extending along the longitudinal axis of the core and having a depth in a direction perpendicular to said longitudinal axis. By "visible" it is herein intended clearly visible by naked eye. Typically the channels have a width generally greater than 1 mm, preferably from 5 mm to 50 mm, more preferably from 6 mm to 40 mm, more preferably from 8 mm to 30 mm, even more preferably from greater than 8 mm to less than 25 mm. The one or more channels are arranged within the inner periphery of the absorbent core, i.e. the inner, or interior, space defined by the periphery of the absorbent core, such that each of the channel(s) is bounded, or surrounded, by absorbent material. Additionally, the length of the channels may be from 10 % to 95 % of the length of the absorbent core so that again the one or more channels are bounded by absorbent material.

"Comprise", "comprising", "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. The absorbent article includes a chassis and may also include other components, such as liquid wicking layers, liquid intake layers, liquid distribution layers, transfer layers, barrier layers, wrapping layers and the like, as well as combinations thereof. Disposable absorbent articles and the components thereof can operate to provide a skin-facing surface and a garment-facing surface.

The "absorbent core" or "absorbent body" as used herein are synonymous and correspond to the absorbent structure disposed between the topsheet and the backsheet of the chassis and is capable of absorbing and retaining liquid body exudates. The size and the absorbent capacity of the absorbent body should be compatible with the size of the intended wearer and the liquid loading imparted by the intended use of the chassis. Further, the size and the absorbent capacity of the absorbent body can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent body. It can contain embossed channels, channels substantially free of material, channels with lower basis weight than the rest of the body, etc.

The term "backsheet" refers to a material forming a liquid impervious cover of the chassis of the absorbent article. The back sheet prevents the exudates contained in the absorbent body from wetting articles such as bedsheets and overgarments which contact the disposable absorbent article. The back sheet may be a composite layer composed of multiple components assembled side-by-side or laminated.

The term "conduit", "duct", "pipe" as used herein are synonyms and are interchangeable and mean a tube or a passageway for conveying a fluid e.g. air, said conduit, duct or pipe being defined by a housing or wall(s) defining an internal volume in which said fluid flows through. Similarly, "convey", "transport", "guide" are synonymous verbs and are interchangeable and mean that are moved, directed or conducted from one point to another.

The term "deflected" as used herein means deviated, ricocheted, rebounded off a surface, that has changed direction after hitting something, that has an obstacle in its course and is forced to change trajectory, that is caused to change direction or that is turn aside from a straight course. Preferably, this term is used herein as a significant change of direction or trajectory. The same definitions applies to "deflecting". In opposition the term "undeflected" means that does not significantly change direction, that is not or slightly deviated.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

By "fluidically connected" as used herein in reference to two elements is meant that a fluid, e.g. air, can circulate between these two elements. For example, two fluidically connected ducts means that a fluid can flow from one duct to the other.

The terms "fluff pulp", "pulp fibers", "fluff", "pulp", "cellulosic fibers" and "absorbent fibers" as used herein are all synonymous and interchangeable and generally reference to an absorbent material, preferably made out of fibers such as cellulosic fibers or any hydrophilic and absorbing fibers.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less.

The terms "inlet", "outlet", "upstream" and "downstream" are with respect to the flow direction of a fluid, e.g. an airflow.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount).

A layer can be liquid and air permeable, permeable to air but impermeable to liquids, impermeable both to air and liquid, or the like. When used in the singular, it can have the dual meaning of a single element or a plurality of elements.

As used herein, the terms "longitudinal", "transversal" and "vertical" are with respect to the apparatus suitable for forming moulded absorbent material deposit structures to be used as absorbent cores for absorbent articles in an assembled state or installed state, said apparatus comprising the apparatus suitable for distributing superabsorbent particles homogeneously onto a substrate. The term "in the direction" as used herein means along that direction, e.g. in the longitudinal direction means along the longitudinal direction.

The terms "mesh", "foraminous forming screen" or "mesh substrate" as used herein are synonymous and mean a screen, a net, a material made of a network of wire(s) or thread(s), an interlaced structure or a weblike pattern or construction, all that have small openings to let air pass through while retaining, or blocking other material such as absorbent material, nonwoven material, or in other words that is air permeable.

The term "nonwoven substrate/material/web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The terms "outlet nozzle" and "outlet section" as used herein are synonymous and interchangeable and refer to the same element, i.e. the extremity of the discharge pipe, namely the extremity of the discharging pipe arranged within the discharging conduit and enabling a crossflow of sub-streams.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "section" as used herein means a segment, a part, a portion of, a division or a component of an element. For example a section of a pipe means a portion of that pipe.

Use of the term "substrate" includes, but is not limited to, woven or nonwoven webs, porous films, ink permeable films, paper, composite structures, or the like.

The term "superabsorbent particle(s)", "superabsorbent polymer(s)", "SAP" and "superabsorbent polymer particle(s)" are interchangeable and all refer to a superabsorbent material suitable for use in the present disclosure are known to those skilled in the art, and may be in any operative form, such as particulate form and mixtures thereof. Generally stated, the "superabsorbent particles" can be a water-swellable, generally water-insoluble, hydrogel-forming polymeric absorbent material, which is capable of absorbing at least about 15, suitably about 30, and possibly about 60 times or more its weight in physiological saline (e.g., saline with 0.9 wt. % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system and may optionally be employed in other components or portions of the absorbent article. The superabsorbent material may be included in the absorbent layer or other fluid storage layer of the absorbent article of the present invention in an amount up to 10% by weight. Typically, the superabsorbent material, when present, will be included in an amount of from about 30% to about 70% by weight, based on the total weight of the absorbent layer. In addition, "superabsorbent particles", "superabsorbent polymer particles" or "SAPs" refer to water-swellable, water-insoluble organic or inorganic materials capable, under the most favorable conditions, of absorbing at least about ((10)) times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between ((10))0 to 800 µm, preferably between 300 to 600 µm, more preferably between 400 to 500 µm.

The term "topsheet" refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven.

Embodiments according to the disclosure will now be described. It is understood that technical features described in one or more embodiments may be combined with one or more other embodiments without departing from the intention of the disclosure and without generalization therefrom.

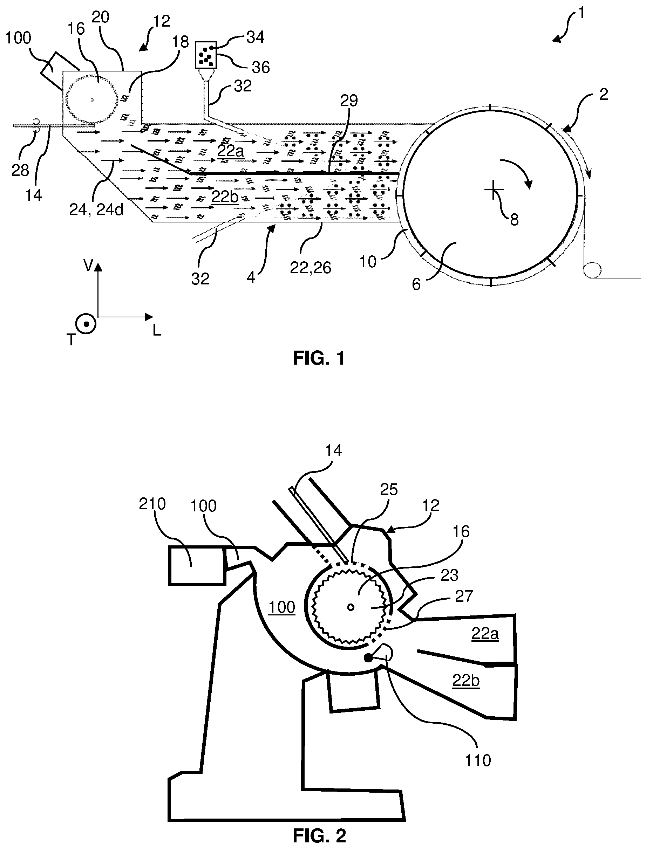

An apparatus 1 apparatus suitable for forming moulded absorbent material deposit structures to be used as absorbent cores for absorbent articles is illustrated in FIG. 1. In other words, FIG. 1 represents a schematic longitudinal cross-sectional view of this apparatus 1 for manufacturing a continuous absorbent body. The apparatus 1 comprises a forming unit 2 and a feeding unit 4. The forming unit 2 comprises a rotary forming drum 6, also called a drum former, that rotates in a circumferential direction about an axis 8, and comprises an outer circumferential surface. A plurality of forming pockets 10 are arranged on the outer circumferential surface of the forming drum 6. Each forming pocket 10 defines a mould cavity where absorbent material is deposited therein thereby forming a moulded absorbent material deposit structure that can be used as an absorbent core for absorbent articles. Each forming pocket 10 comprises a mould cavity being bounded by at least one exterior surface, or by a masking component, defining the contour of the absorbent core to be formed and a bottom air permeable base surface. The bottom air permeable base surface comprises a foraminous forming screen, or mesh substrate, adapted to suck air while maintaining, or retaining, the absorbent material deposited from the feeding unit 4 thereon. The forming pocket 10 can optionally comprise a three-dimensional shaping profile member, or a channel insert, within the mould cavity to form a moulded absorbent material deposit structure, *i.e.* an absorbent core, with channels. For example, the forming pocket 10 can comprise a base or frame, supporting a mesh substrate, or foraminous forming screen, a masking component defining the outer shape of the absorbent core and a channel insert. The foraminous forming screen, the masking component and the channel insert defining the mould cavity where the absorbent material is deposited. The absorbent body formed can be continuous or discontinuous depending on the forming pocket 10 used, on the absorbent material used, if the absorbent core is wrapped *etc.*

The absorbent material refers to the material constituting the absorbent core of the absorbent article. Examples of commonly occurring absorbent materials are selected from and not limited to cellulosic fluff pulp, tissue layers, highly absorbent polymers or superabsorbent polymer particles (SAP), absorbent foam materials, absorbent nonwoven materials or the like. For example, the absorbent core comprises cellulosic fluff pulp and/or superabsorbent particles, i.e. SAP.

The feeding unit 4 comprises a defibrating station 12 that defibrates a continuous sheet of absorbent material 14 such as a sheet of cellulose material into fresh absorbent fibers 18. "Fresh" as used herein means as recently made or obtained, hence "fresh absorbent fibers 18" signifies within the present disclosure the absorbent fibers 18 formed by the defibrating machine 16 or freshly defibrated absorbent fibers 18. The defibrating station 12 comprises: a defibrating machine 16, or defibrating device, that defibrates the sheet of absorbent material 14 thereby producing a plurality of fresh absorbent fibers 18, or pulp fibers, or cellulosic fibers, fluff or fluff pulp and a defibration station housing 20 that covers, or houses, the defibrating machine 16. The defibrating station 12 can also include a pair of feed rollers 28 that supplies the sheet of absorbent material 14 to the defibrating machine 16. The defibrating machine 16 is a device breaking the absorbent material sheet 14 into smaller pieces by grinding, crushing, or cutting, it can be for example a mill such as a hammermill.

As explained hereabove, the feeding unit 4 comprises a defibrating station 12. The defibrating station 12 is configured to generate absorbent fibers 18 and a first airflow 24. The defibrating station 12 comprises a defibrating machine 16 suitable for defibrating a sheet of pulp material 14 and for generating absorbent fibers 18 and a first airflow 24. The defibrating station also comprises a recycling duct 100 by-passing the defibrating machine 16 and conveying recycled absorbent fibers 18 from a filtering apparatus 210 that is described hereunder. According to the present disclosure, the defibrating machine 16 and the recycling duct 100 are fluidically connected to at least two ducts 22, also called a discharging conduit 22 guiding the absorbent fibers from the feeding unit 4 to the forming unit 2, said discharging conduit 22 is further described hereunder. In other words, the defibrating machine 16 and the recycling duct 100 are both fluidically connected to at least two ducts 22. The defibrating machine 16 and the recycling duct 100 are both fluidically connected to two ducts 22a,22b, or two discharging conduit 22a,22b. For example, as illustrated in FIG. 2, the defibrating machine 16 and the recycling duct 100 are both fluidically connected to two ducts 22a,22b, meaning the defibrating machine 16 is fluidically connected to two ducts 22a,22b and the recycling duct 100 is also fluidically connected to said two ducts 22a,22b.

The defibrating station 12 is connected, meaning fluidically connected, to a discharging conduit 22 through which a first airflow 24 flows, in a first airflow direction 24d. The first airflow 24 is generated by the rotation of the defibrating machine 16 and transports the absorbent fibers 18 produced in the defibrating station 12, within the discharging conduit 22. In other terms, the absorbent fibers 18 are conveyed by the first airflow 24 along the discharging conduit 22. The discharging conduit 22 corresponds to a duct defined by a housing 26 in which the first airflow 24 and the absorbent fibers 18 flow. In other terms, the housing 26 defines an internal or inner volume, i.e. the discharging conduit 22, where the first airflow 24 and absorbent fibers 18 circulate. The discharging conduit 22 fluidically connects the defibrating station 12 and the forming unit 2. In particular, the discharging conduit 22 comprises at least one inlet fluidically connected to the outlet of the defibrating station 12 and the discharging conduit 22 comprises at least one outlet fluidically connected to the inlet of the forming unit 2, meaning that one end of the discharging conduit 22 covers at least partially the outer peripheral surface of the forming drum 6. As illustrated in FIG. 1, the discharging conduit 22 comprises a inner wall 29 defining two ducts 22a,22b, or two sub-ducts 22a,22b, the two ducts 22a,22b fluidically connecting the defibrating station 12 and the forming unit 2. In other words, the inner wall 29 divides at least partially the discharging conduit 22 into two ducts 22a,22b. As illustrated in FIG. 2, the discharging conduit 22 comprises two separate, or distinct, ducts 22a,22b, the two ducts 22a,22b fluidically connecting the defibrating station 12 and the forming unit 2. The present disclosure is not limited to these embodiments, the discharge conduit 22 can comprise only one single duct to convey the absorbent fibers 18 and first airflow 24 from the defibrating station 12 to the forming unit 2. The discharge conduit 22 can comprise three ducts or four ducts or more to transport the absorbent fibers 18 and first airflow 24 from the defibrating station 12 to the forming unit 2.

As explained hereabove, the defibrating station 12, namely the defibrating machine 16 generates the first airflow 24. The defibrating machine 16 is a device which takes in a sheet of absorbent material 14 and shreds it into fibers with a rotating defibrating element 16. The defibrating machine 16, e.g. a hammermill, namely the rotating element, generates an air stream that flows within the discharging conduit 22 to the forming drum 6. In other words, the defibrating machine 16 generates the first airflow 24 with a volumetric flow rate (m³.s⁻¹) comprised between 0,3 and 6 m³.s⁻¹, preferably between 1 and 4 m³.s⁻¹, depending on the rotation speed (RPM) of the hammermill that is comprised between 2000 and 5000 RPM (rotation per minute), preferably between 2500 and 3500 RPM. The hammermill 16 is arranged in a drum housing 23 comprising an inlet 25 through which the absorbent material 14 and an airflow can enter and an outlet 27 through which the shredded absorbent fibers 18 and first airflow 24 can flow out of, the recycling duct 100 matches the curvature of the drum housing as illustrated in FIG. 2. The at least one discharging conduits 22,22a, 22b defined at least partially by a housing 26 fluidically connects the outlets 27,100 of the defibrating station 12, meaning the outlet 27 of the drum housing 23 and the outlet of the recycling duct 100, and the forming unit 2 through which the plurality of absorbent fibers 18 and a first airflow 24 flow. The at least one flap 110 can be placed within the recycling duct 100 only or at the outlets 27,100 of the defibrating station 12.

The recycling duct 100 comprise at least one flap 110 to convey the recycled absorbent fibers into one, two or more ducts 22a,22b as illustrated in FIG. 2. As illustrated in FIG. 2, the flap 110 can obturate the duct 100 entirely, meaning a closed position so that only newly crushed pulp is guided toward the forming unit 2. The flap 110 can also be pivoted so as to offer as little resistance as possible to the flow absorbent fibers coming from the filtering apparatus 210 meaning an open position where the recycling ducts is not or barely obturated. The flap 110 can be positioned into any intermediate position between said open and closed positions The flap 110 can also be oriented in a way that the recycled absorbent fibers are guided toward only one duct 22a and not the other 22b. The flap can be a door flap, a butterfly flap, a drum flap, a sliding door flap or any kind of conventional air flap. Said flap comprising an actuator to control its rotation and pivoting. The flaps are arranged to pivot or rotate between a position to obstruct a duct and a position where is oppose a minimal resistance to the flow of air. For example, FIG. 2 shows a recycling duct 100 comprising a drum flap. The apparatus can comprise one discharging conduit 22 or two ducts 22a,22b. The at least one flap 110 can also be placed at the outlet of the discharging station 12 to guide the flow of fresh and/or recycled absorbent fibers 18 withing one, two or more ducts 22,22a,22b,22c. There can be one or more flaps dedicated to the recycling duct 100 and/or one or more flaps dedicated to the outlet 27 of the drum housing 23 of the defibrating machine 16.

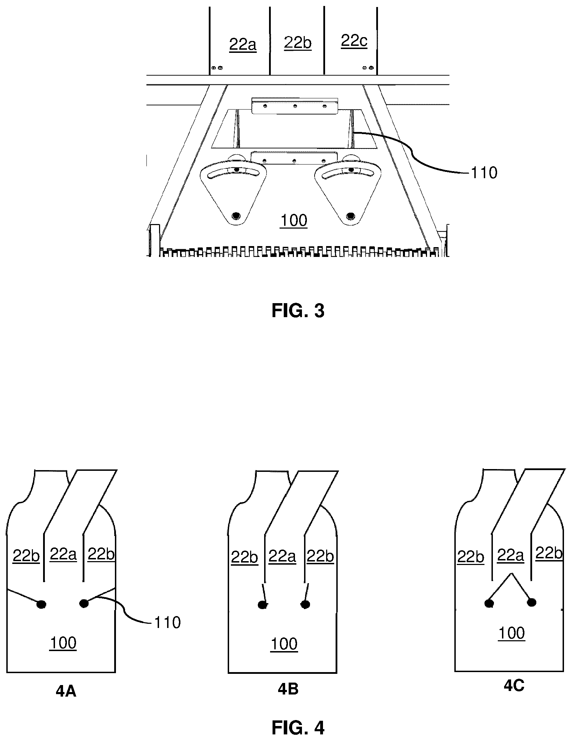

As illustrated in FIG. 3, the apparatus can comprise three ducts 22a,22b,22c in fluidically connection with the recycling duct 100 and/or outlet 27 of the drum housing 23 of the defibrating machine 16. The apparatus 1, preferably recycling duct 100, comprises two flaps 110 to convey the recycled absorbent fibers into two or all three ducts 22a,22b,22c.

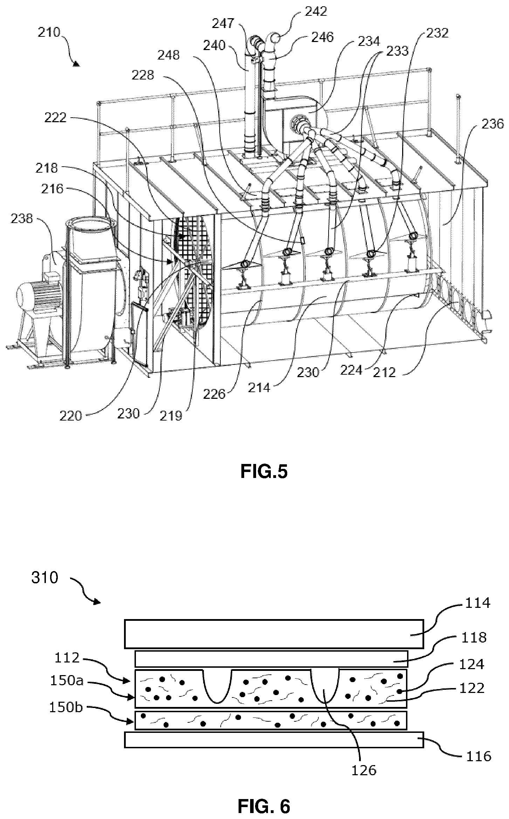

As illustrated in FIG. 4, the apparatus comprises two ducts 22a,22b in fluidically connection with the recycling duct 100 and/or outlet 27 of the drum housing 23 of the defibrating machine 16. The apparatus 1, preferably the recycling duct 100, comprises two flaps 110 to convey the recycled absorbent fibers into one or both ducts 22a,22b. The duct 22a comprises only one inlet linked to the recycling duct 100, whereas the duct 22b comprises two inlets linked to the recycling duct 100, the two inlets rejoining downstream. The flaps 110 are illustrated here as door claps and in conformation 4A are arranged to guide the recycled absorbent fibers towards one duct, the duct 22a by blocking the two inlets of duct 22b. In conformation 4B, the flaps 110 are arranged to guide the recycled absorbent fibers towards both ducts, the ducts 22a and 22b, by orienting the recycled absorbent fibers towards all three inlets. In conformation 4C, the flaps 110 are arranged to guide the recycled absorbent fibers towards one duct, the duct 22b, by blocking the inlet of duct 22a. As illustrated in FIG. 1, the duct 22a is the upper duct with respect to the vertical direction, and provides the absorbent material for the top layer 150a of the absorbent core 112, whereas the duct 22b is the lower duct with respect to the vertical direction, and provides the absorbent material for the bottom layer 150b of the absorbent core 112 (cf. FIG. 6).

The forming drum 6 is connected to a suction fan (not shown) through a suction duct (not shown). The suction fan is driven so that the pressure at a prescribed portion inside the forming drum 6 is kept negative. A substrate such as a now-woven web, e.g. a core wrap, or absorbent material can be applied on the mesh substrate, or foraminous forming screen, and be maintained on said foraminous forming screen with the negative pressure. The absorbent material such as SAP or fluff is deposited onto said non-woven substrate thereby forming a congregate of absorbent material suitable to be used as an absorbent core for an absorbent article. The absorbent material is maintained on the substrate and/or foraminous forming screen by the negative air-pressure generated by the suction, the pores formed in the mesh substrate functioning, or serving or acting, as suction holes. Moreover, the intake of air from the pores partially generates the first airflow 24. In other words, the suction fan generates a portion of the first airflow 24 within the discharging conduit(s) 22, the pulp fibers 18 generated by the defibrating machine 16 are dispersed within the first airflow 24 and are guided to the forming drum 6. The outlet of the discharging conduit(s) 22 is arranged in such a way that it covers at least a portion of the outer circumferential surface of the forming drum 6 and the forming pockets 10 arranged onto the outer circumferential surface of the forming drum 6. The suction fan further promotes the circulation of the first airflow 24. Hence, the absorbent fibers 18 are guided by the first airflow 24 throughout the discharging conduit(s) 22 from the defibrating station 12 to the mould cavity arranged within the forming pocket 10. As explained hereabove, the mould cavity comprises a mesh substrate so that the absorbent fibers are retained within the mould cavity to form a moulded absorbent material deposit structure, *i.e.* an absorbent core, the mesh substrate comprising micro-pores to let air pass through while retaining bigger objects such as fibers or super absorbent particles or a non-woven web.

As explained, hereabove, the absorbent material can also comprise superabsorbent particles (SAP). The absorbent core can comprise a combination of cellulosic fibers, i.e. fluff and superabsorbent particles. The absorbent core can comprise a weight ratio between SAP against fluff comprised between 20/80 to 80/20 (% wt.). The absorbent core can also be essentially fluff-less meaning that the absorbent core contains less than 20% by weight of fluff pulp, for example less than 10% fluff pulp, less than 5% fluff pulp but more than at least 1% as the object of the present disclosure is to use recycled fluff. The absorbent core can also be essentially SAP-less meaning that the absorbent core contains less than 20% by weight of SAP.

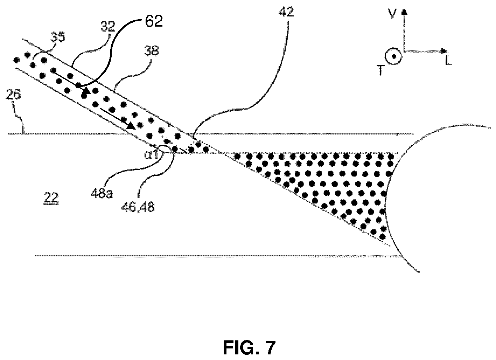

As illustrated in FIG. 1, the feeding unit 4 of the apparatus 1 for forming moulded absorbent material deposit structures to be used as absorbent cores for absorbent articles comprises an apparatus suitable for distributing superabsorbent particles homogeneously onto a substrate, this substrate being the mould cavity of a forming pocket 10 eventually already comprising a nonwoven fabric arranged on the foraminous forming screen of the forming pocket 10. The apparatus suitable for distributing superabsorbent particles homogeneously onto a substrate illustrated in FIG. 7 comprises a superabsorbent particle discharge pipe 32 that supplies superabsorbent particles 34 into the discharging conduit 22 or discharging conduits 22a,22b. More particularly, the discharge pipe 32 conveys a stream of superabsorbent particle 34 and a second airflow 62 to the discharging conduit 22 or discharging conduits 22a,22b. The discharging conduit 22 or discharging conduits 22a,22b and the discharge pipe 32 are fluidically connected, with the discharge pipe 32 extending partially within the discharging conduit 22 or within each discharging conduits 22a,22b and comprising an outlet arranged within inner volume of the discharging conduit 22 or discharging conduits 22a,22b.

As illustrated in FIG. 1, the apparatus 1 for forming moulded absorbent material deposit structures extends in a longitudinal direction L (length), in a transversal direction T (width) and in a vertical direction V (height).

The discharge pipe 32 supplying, or discharging, superabsorbent particles 34 can be arranged in the upper part of the discharging conduit(s) 22 or alternatively in the lower part of the discharging conduit(s) 22 with respect to the vertical direction V. This discharge pipe 32 in conjunction with a second airflow 62 (FIG. 7), which has, or flows in, a first direction, supplies the SAP 34 and air into the discharging conduit(s) 22. In other words, the superabsorbent particles 34 guided, or transported, in the discharge pipe 32 are dispersed within the discharging conduit(s) 22 and are guided to the forming drum 6. The discharge pipe 32 guides, or conducts, a stream 35 comprising superabsorbent particles 34 and a second airflow 62. Hence, the superabsorbent particles 34 are guided by the first and first airflow 24 from a reservoir 36 such as a bag or container to the mould cavity arranged within the forming pocket 10. As explained hereabove, the mould cavity comprises a mesh substrate so that the superabsorbent particles 34 and/or absorbent fibers 18 are retained within the mould cavity to form a moulded absorbent material deposit structure, *i.e.* an absorbent core, the mesh substrate comprising micro-pores to let air pass through while retaining bigger objects such as fibers and/or super absorbent particles. The same applies when the apparatus comprises two discharging conduit 22a,22b.

FIG. 7 illustrates a portion of the discharging conduit 22 defined by the housing 26 and a portion of the discharge pipe 32 which partially extends within the discharging conduit 22. The discharging conduit 22 comprises here a transversal cross section that is rectangular. The discharge pipe 32 comprises a conveying section 38 and an outlet section comprising an outlet nozzle. The outlet nozzle comprises a straight portion 42 arranged in the continuity of the conveying section 38 and a deflecting portion 46. The deflecting portion 46 comprises deflecting means 48 arranged in a way that only a portion of the stream of superabsorbent particles 34 and second airflow 62 are deflected and the other portion of said stream of superabsorbent particles 34 and second airflow 62 is not deflected by the outlet nozzle. In other words, the outlet nozzle comprises convergent portion 46 and a flat portion 42, the flat portion 42 being neither divergent nor convergent.

For sake of clarity, it is to be understood that the discharge pipe 32, in particular the conveying section 38, extends in a longitudinal direction L (length), in a transversal direction T (width) and in a vertical direction V (height), the outlet nozzle is arranged in such a way that the straight portion 42 extends in the same directions (L,T,V) as the conveying section 38 whereas the deflecting portion 46 extends in at one in one direction that is not rectilinear to the directions in which the conveying section 38 extends in. In other words, the straight portion 42 and the conveying section 38 extend in a same plane whereas the deflection portion 46 and the conveying section 38 extend in different planes. According to the embodiment as illustrated in FIG. 7, the deflecting portion 46 does not extend in the vertical direction or at least does not extend in the same vertical direction as the conveying section 38, or in other words, the deflecting portion 46 extends only in a plane defined by the longitudinal and transversal directions unlike the conveying section.

As illustrated in FIG. 7, the outlet section, meaning the outlet nozzle comprises an inlet, meaning a proximal end, which is coupled, or linked or forms a continuity of matter with, to the conveying section 38 of the discharge pipe 32, and an outlet meaning a distal end and also the outlet of the discharge pipe 32, which is arranged inside the discharging conduit 22. The outlet is the frontier between the inner volumes of the discharge pipe 32 and the discharging conduit 22. The outlet nozzle has a rectangular cross-sectional area along its length and comprises a top wall corresponding to the straight or flat portion 42, a bottom wall corresponding to the deflecting portion 46 or deflecting means 48 and a first and second side wall. In other words, the outlet nozzle comprises a rectangular transversal cross section and the cross-sectional area of outlet nozzle is lesser than the cross-sectional area of the conveying section. For example the cross-sectional area of the conveying section is 3600 mm² whereas the cross-sectional area of the outlet nozzle at the distal end is 2700 mm², or in other words, the cross-sectional area for the passage of the second airflow 62 and superabsorbent particles 34 is reduced by between 10 % and 40%, preferably by between 15% and 30%, for example by 25%. Proximal and distal are used herein in reference to the conveying section 38 of the discharge pipe 32, proximal being the point of the outlet nozzle closest to the conveying section 38 and distal being the point of the outlet nozzle farthest from the conveying section 38. The top wall, or straight portion 42, comprises a proximal end and a distal end. Both proximal and distal ends of the straight portion 42 are aligned with the conveying section 38 meaning that these ends are rectilinear with any point of the conveying section 38, meaning defining an angle of 180°. The bottom wall, or deflecting portion 46, comprises a proximal and a distal end. The distal end of the deflection portion 46 is unaligned with the bottom wall of the conveying section 38, or in other words, any point of the conveying section, the proximal end and the distal end of the deflection portion 46 are not aligned, meaning non-rectilinear thereby defining the first angle α1. Said first angle α1 is comprised between 91° and 179°, preferably between 120° and 175°, more preferably between 150° and 170°.

The apparatus for manufacturing a continuous absorbent body comprises a first covering unit that supplies a first endless substrate precursor, preferably a nonwoven web, that covers the plurality of forming pockets 10 of the forming drum 1, before providing the homogeneous mixture of pulp fibers and SAP in the forming pockets 10. After placing the homogeneous mixture of absorbent fibers 18 and SAP 34 on the first endless substrate precursor, folding guide plates (not shown) fold both lateral sides of the first substrate precursor in the width direction to cover the mixture absorbent fibers 18 and SAP 34 thereby defining a continuous wrapped absorbent body.

The absorbent article 310 comprising absorbent cores 112 formed by the apparatus and process as described herein comprises an absorbent core 112 arranged between a liquid permeable topsheet 14 and a liquid impermeable backsheet 116. An optional acquisition distribution layer 18 can be positioned between said topsheet 114 and said absorbent core 112. The absorbent core 12 comprises absorbent material selected from the group consisting of cellulose fibers 18, superabsorbent polymers 34 or a combination thereof. The absorbent core 112 can comprises at least one channel 126 substantially free of cellulose fibers 18 and/or substantially free of superabsorbent polymer 34. The topsheet 14 is associated to the backsheet 16. The topsheet 14 can be directly joined to backsheet 16 by affixing the topsheet 14 directly to the backsheet 16. Alternatively, the topsheet 14 can be indirectly joined to the backsheet 16 by affixing the topsheet 14 to intermediate members which in turn are affixed to the backsheet 16.

The apparatus enables to form absorbent cores 112 that can comprise a single layer of absorbent material or a plurality of layers as exemplified in FIG.6. In the embodiment illustrated in FIG. 6, the absorbent core 112 comprises two distinct absorbent core layers 150a,150b. The first core layer 150a , or top layer, comprises a first concentration of super absorbent polymer 124a therein and a second core layer 150b, or bottom layer, comprises a second concentration of super absorbent polymer 124b therein said first and second concentrations being different. Preferably, the second concentration of super absorbent polymer is greater than the first concentration of super absorbent polymer, preferably wherein said second concentration is at least 1.5, preferably 2, times greater than the first concentration. Such brings advantages such as reduced risk of gelblocking. The single layer of the absorbent core 112 or the first absorbent core layer 150a (FIG. 6) comprises one or more channels 126, said channel(s) 126 being continuous at least along the length and the width of said absorbent core 12. Both layers 150a, 150b can comprise one or more channels 126, or only the bottom layer 150b can comprise one or more channels 126.

When the absorbent core 112 comprises two layers of absorbent material 18,34 as illustrated in FIG. 6. The feeding unit 4 comprises in this case two discharging conduits 22a,22b linked to the defibrating station 12. The feeding unit 4 comprises two discharge pipes 32a,32b as described herein, each with an outlet nozzle 40 as described herein. The two layers can be identical, or they can comprise different amounts of absorbent fibers 18 and/or superabsorbent particles 34. For example, if the lower discharge pipe 32a comprises a blower functioning at a lower rotational speed than the blower comprised in the upper discharge pipe 32b, e.g. 1500 RMP and 1800 RPM respectively, then the amount of SAP will be lesser in the lower layer of the absorbent core. According to another example, both discharge pipe 32a,32b exploit one air-generating device that is fluidically connected to both pipes, but the venturi section in said pipes is different, e.g. placing an obstacle in one pipe or having pipes of different dimensions, hence leading also to the layers of the absorbent core having different amount of SAP 34. Of course, it is possible to make absorbent cores 112 comprising only one layer of absorbent material with an apparatus still comprising two conduits 22a,22b.

The method for manufacturing a continuous absorbent core using an apparatus as described herein. For sake of clarity said apparatus comprises: a forming unit 2 and a feeding unit 4; the forming unit 2 having: a forming drum 6 comprising an outer circumferential surface, a plurality of forming pocket 10 being arranged on the outer circumferential surface of the forming drum 1, said forming pocket 10 comprising a mould cavity defined by a foraminous forming screen and eventually a masking element disposed within the pockets 10; and the feeding unit 4 comprising a defibrating station 12 with a defibrating machine 16, such as a hammermill, a discharging conduit 22 through which a first airflow 24 flows, a superabsorbent polymer discharge pipe 32 through which a second airflow 62 flows comprising an outlet nozzle 40 as described herein. The method for manufacturing a continuous absorbent body comprises;
- a defibrating step wherein a continuous sheet of absorbent material 14 is supplied and defibrated by the defibrating machine 16 at the defibrating station 12, thereby obtaining a plurality of absorbent fibers 18;
- a conveying step where a stream of recycled absorbent fibers 18 is transported by the recycling duct 100;
- a transport step wherein said plurality of absorbent fibers 18 and/or recycled absorbent fibers 18 are transported, or conveyed or guided, by the first airflow 24 which generated by the defibrating machine 16 and the suction fan arranged in the forming unit 2, said fibers 18 are guided from the defibrating station 12, through the first airflow 24 circulating inside the discharging conduit 22 to the forming unit 2 and the forming pocket 10;
- a feeding step wherein a specific amount of superabsorbent particles 34 in conjunction with the second airflow 62 is sprayed through the superabsorbent polymer discharge pipe 32 and the outlet nozzle 40 into the discharging conduit 22 via the outlet nozzle 40, said outlet nozzle creating a crossflow of superabsorbent particles 34,
- eventually a mixing step, wherein the plurality of absorbent fibers 18 transported by the first airflow 24 are mixed with the crossflow of SAP sprayed by the discharge pipe 32 and the outlet nozzle 40; and
- an accumulation step wherein the absorbent fibers 18 and the superabsorbent particles 34 accumulate within the mould cavity of the forming pocket 10 thereby forming an absorbent core structure.

As written hereabove, according to an embodiment, it is possible to make fluff-less absorbent core, meaning that the hereabove method can only comprise the spraying of superabsorbent particles 34 within the mould cavity as described herein.

In an additional embodiment, the method can comprise an additional step, a covering step performed prior to the accumulation step, in which a covering substrate precursor, in other words a core wrap, such as a non-woven material, is applied to cover the mould cavity of forming pockets 10. Subsequently, the accumulation step is carried out where the absorbent material, meaning the absorbent fibers 18 and the superabsorbent particles 34, is deposited into the mould cavity and onto the covering substrate precursor. After the accumulation step, a folding step is carried out where the covering substrate precursor is folded over the mixture of pulp fibers and superabsorbent particles 34, thereby defining a wrapped absorbent core. Alternatively, instead of folding over the covering substrate precursor, the method can comprise a second covering step where a second covering substrate, in other words a second core wrap, such as a layer of nonwoven material, is applied to cover the mixture of pulp fibers and superabsorbent particles 34 thereby defining a wrapped absorbent core.

As seen previously, the forming pocket 10 and mould cavity can comprise a channel insert, the method can comprise a further pressing step, where pressing means, such as one or more rollers, apply pressure onto the wrapped absorbent core to ensure that the core wraps are properly joined, or bonded, together at the periphery and/or at the central area thereby ensuring a proper sealing at the periphery and/or ensuring a proper formation of channel(s). Adhesive can be applied on the core warp(s) to strengthen the bonding.

The method can also comprise a removal step, where a surplus of absorbent material can be removed by removal means, such as a brush with bristles or a blower, from the mould cavity and/or channel insert to have a uniform amount of absorbent material in the absorbent cores, said removal step being after the accumulation step and before the covering step.

In an additional embodiment, namely if the absorbent core comprises two layers of absorbent material, the method can comprise as an accumulation step:
- a first covering step in which a first covering substrate precursor, meaning a bottom core wrap, such as a non-woven material, is applied to cover the mould cavity of forming pockets 10,
- a first accumulation step, in which the absorbent material, meaning the pulp fibers and superabsorbent particles 34, is deposited into the mould cavity and onto the first covering substrate precursor;
- a second covering step, in which a second covering substrate, meaning an intermediate core wrap such as a non-woven material, is applied to cover the absorbent material,
- a second accumulation step in which absorbent material is deposited into the mould cavity and onto the second covering substrate; and
- a third covering step in which a third covering substrate, meaning a top core-wrap such as a non-woven material, is applied to cover the absorbent material, thereby defining a wrapped absorbent core comprising two layers of absorbent material.

The filtering apparatus 210 for filtering air from an absorbent articles manufacturing process line, said filtering apparatus 210 comprises as illustrated in FIG. 5:
- a housing 236 comprising at least one inlet;
- a filtering media 214 arranged downstream with respect to the airflow of the at least one inlet ;
- a support structure 216 comprising a cylindrical drum 218 comprising a first and second longitudinal end, the filtering media 214 being secured to said cylindrical drum 218;
- a motor device to rotate the cylindrical drum 218; and
- a first pump 238.

According to the disclosure, the motor device comprises a driving mean and the cylindrical drum 218 comprises the corresponding driven mean arranged at the periphery of one of the longitudinal ends of the cylindrical drum 218, the driving mean actuating the driven mean and cylindrical drum 218.

The air filtering apparatus 210 preferably comprises a plurality of inlets 212 or air inlets, for example five air inlets 212 as illustrated in FIG.5. The disclosure is not limited to this number of inlets 212. The filtering apparatus 210 may comprise two, three, four, six, seven, eight, nine, ten or more inlets 212.

The filtering apparatus 210 comprises a support structure 216 to carry, support or maintain, the filtering media 214 in a steady position, the filtering media 214. For example, the support structure 216 comprises a cylindrical drum 218 elongated, or extending, in a longitudinal direction and comprising a grid 219, latticework or mesh 219 that can retain the filtering media 214 while being air-permeable. The mesh 219 defines the lateral surface of the cylindrical drum 218. The cylindrical drum 218 can further comprise a central shaft 220 around which the cylindrical drum 218 rotates and optionally several reinforcements 222 such as rods, or arms, linking the central shaft 220 to the cylindrical drum 218 or bridging two diametrically opposed points of the cylindrical drum 218. The reinforcements 222 can extend in the radial direction and/or in the longitudinal direction of the cylindrical drum 218. The filtering media 214 is fastened, or secured, onto the support structure 216. The filtering media 214 can for example comprise a zipper system 224 comprising a slider connecting two transversal ends of the filtering media 214 around the cylindrical drum 218, the transversal ends comprising two rows of teeth that are designed to interlock thereby joining the transversal ends together around the cylindrical drum 218. The filtering media 214 can also be fastened onto the cylindrical drum 218 with straps 226 and corresponding buckles 228. As illustrated in FIG. 1, the air filtering apparatus 210 comprises here five straps 226 to further secure the filtering media 214 to the cylindrical drum 218 in addition of the zipper 224.

The central shaft 220 when present can extend along the entire length of the cylindrical drum 218, or the central shaft can extend along a portion of the longitudinal axis of the cylindrical drum 18.

The filtering apparatus 210 may also comprise a frame 230 to maintain the support structure 216. The frame 230, or space frame, comprises a plurality of struts and/or reinforcements such as rods or arms forming a truss-like structure to carry and support the support structure 216, in particular the cylindrical drum 218.

The air filtering apparatus 210 comprises at least one nozzle 232, for example, two, three, four, five, six, seven, eight, nine, ten or more nozzles 232, here five nozzles are illustrated in FIG.1. The one or more nozzles 232 are linked through ducts 233 to a pump 234, that will be referenced as the recycling pump 234 henceforth, enabling a suction of air and absorbent material contained in said air. The plurality of nozzles 232 are arranged in close proximity to the filtering media 214. More precisely, the recycling pump 234 is arranged and configured to suck up a portion, meaning not the entirety, of the air incoming in the housing 236.

Each nozzle 232 comprises an inlet which is preferably convergent with respect to the airflow direction where the cross section of the nozzle is narrowing down, or decreasing, from a greater cross section to a lesser cross section in the direction of the flow. By being convergent and in close proximity of the filtering media 214, the surface of suction of the nozzle 232 is greater thereby increasing the amount of absorbent material to be suctioned by the recycling pump 234. In other words, the greater cross section 232 of the nozzle is closest to the filtering media 233 with respect to the airflow direction.

The air filtering apparatus 210 also comprises a housing 236 defining an inner volume with a set of walls, for example four walls and a ground wall and ceiling wall and optionally partition walls within said inner volume. The filtering media 214, support structure 216, nozzles 232 and frame 230 are arranged at least partially within said inner volume. The housing 236 enables to contain the air to be filtered within said inner volume and directs the airflow to pass through the filtering media 214. The air filtering apparatus 210 also comprises a pump 238, that will be referenced as the main pump 238 henceforth, enabling a suction of air and absorbent material contained within said air. The main pump 238 is preferably arranged downstream of the filtering media 214 with respect to the airflow. The main pump 238 comprises an inlet that is in fluidic connection with the inner volume defined by the housing 236 and an outlet that is arranged outside, or in the exterior of, the housing 236, or at least in fluidic connection with the exterior of the housing.

The main pump 238 and the recycling pump 234 are both means for moving fluids, in this case air. The main pump 238 can be referenced as the first pump 238 and the recycling pump 234 can be reference as the second pump 238. The present disclosure is not limited to the nature of these pumps. They can be selected from and not limited to centrifugal pumps such as centrifugal fans, axial-flow pumps, diaphragm pumps, impeller pumps, screw pumps, booster pumps, canned motor pump, circulator pumps, single or multistage pumps and turbine pumps. The main pump 238 is the pump ensuring the vacuum in the absorbent articles manufacturing process line and has preferably a higher volume flow rate, or air capacity, then the recycling pump 234. In other words, the main pump 238 enables a greater flow than the recycling pump 234, *i.e.* the flow of air passing through the main pump 238 is greater than the flow passing through the recycling pump 234. The volume flow rate, or air capacity, of the main pump 238 is at least ten times greater than the air capacity of the recycling pump 34. Preferably, the volume flow rate, or air capacity, of the main pump 238 is at least twenty times, more preferably between 25 and 50 times greater than the air capacity of the recycling pump 234. For example, the recycling pump 234 has a volume flow rate of about 1000 m³/h whereas the main pump 238 has a volume flow rate between about 30 000 m³/h and about 40 000 m³/h. The main pump 238 and the recycling pump 234 are preferably of the same nature, for example, the main pump 238 and the recycling pump 234 are both centrifugal fans. Alternatively the main pump 238 and the recycling pump 234 can be of different nature. For instance, given that the main pump 238 is destined to move air and the recycling pump 234 is destined to move air and absorbent material, the recycling pump can have specific seals and a impeller adapted to avoid clogging. Given that the main pump 238 has a greater air capacity than the recycling pump 234, the main pump 238 will suck in the majority of the air incoming in the housing 236 and the recycling pump 234, through the nozzle will suck a lesser portion of that air than the main pump 238. In other words, given the arrangement and the air capacities of the pumps 234,238, the majority of the air incoming in the housing 236 flows through the main pump 238 and a minority of that incoming air flows through the recycling pump 234.

Following the airflow, the air coming from the process line and containing absorbent material such as cellulosic fibers e.g. fluff and/or superabsorbent particles, enters the filtering apparatus 210, namely enters within the inner volume defined by the housing 236 through the one or more air inlets 212. The air then passes through the filtering media 214 and is then extracted from the inner volume by the main pump 238. The filtering media 214 filters the air so that the absorbent material (cellulosic fibers and/or SAP) contained in the air is retained within, or on the surface of, the filtering media 214.

The one or more nozzles 232 being placed in close proximity to the filtering media 214 enable to extract said absorbent material from the filtering media 214. The recycling pump 234 comprises one inlet that is linked one or more nozzles 232 and a first and second outlets 240,242. The first outlet 240 is in fluidic connection with the inner volume defined by the housing 236. In other words, the recycling pump 238 can re-inject the air and absorbent material within the air filtering apparatus 210 and ensure a re-filtering of the air. The second outlet 242 is in fluidic connection with the process line, namely with the absorbent core forming part of the process line where the recycled absorbent material can be re-used and arranged within an absorbent article. More specifically, the second outlet 242 is in fluidic connection with the recycling duct 100 by-passing the defibrating machine 16 and conveying recycled absorbent fibers 18 from a filtering apparatus 210 to the ducts 22a,22b and to the forming pocket 10.

The recycling pump 234 or outlet duct of said pump 234 preferably comprises a valve 246 to guide the air through one outlet or the other. When the process line for manufacturing absorbent articles is running, the valve 246 guides the air and absorbent material towards the second outlet 242 to recycle the absorbent material whereas if the process line is temporarily stopped, the valve 246 guides the air and absorbent material toward the first outlet 240 to refilter the air and ensure a closed air loop. The valve 46 is preferably a three-port valve such as a three-way ball valves come with a L- shaped fluid passageways inside the rotor. The valve 246 also comprises an actuator 247 to control the direction of the airflow, preferably the valve 246 comprises an electromechanical actuators such as an electric motor or solenoid to enable an automatic control or a remote control. For instance, when the process line is temporarily stopped, it can send, or emit, an electronic signal to the actuator 247 to orientate the airflow towards the first outlet 240 and housing 236.

## Claims

1. **Apparatus** for distributing absorbent fibers (18) onto a substrate, the apparatus comprising a defibrating station (12) comprising :
a. a defibrating machine (16) suitable for defibrating a sheet of pulp material (14) and generating fresh absorbent fibers (18) and a first airflow (24), and
b. a recycling duct (100) by-passing the defibrating machine (16) and conveying recycled absorbent fibers (18) from a filtering apparatus (210),
wherein the defibrating machine (16) and the recycling duct (100) are fluidically connected to at least one duct (22,22a,22b), wherein the defibrating machine (16) and the recycling duct (100) are fluidically connected to at least two ducts (22,22a,22b), wherein the recycling duct (100) comprises at least one flap (110) to convey the recycled absorbent fibers (18) into one, two or more of the ducts (22,22a,22b).

2. Apparatus for distributing absorbent fibers (18) onto a substrate according to claim 1, wherein the apparatus comprises two ducts (22,22a,22b) and the recycling duct (100) comprises two flaps (110) to convey the recycled absorbent fibers (18) into one or two ducts (22,22a,22b).

3. Apparatus for distributing absorbent fibers (18) onto a substrate according to claim 1 or 2, wherein said at least one flap (110) is selected from a door flap, a butterfly flap, a drum flap, a sliding door flap.

4. Apparatus for distributing absorbent fibers (18) onto a substrate according to any of the preceding claims, wherein the defibrating machine (16) is a hammermill arranged in a drum housing (23), the recycling duct (100) following the curvature of said drum housing.

5. An apparatus (1) suitable for forming moulded absorbent material deposit structures to be used as absorbent cores for absorbent articles, the apparatus (1) comprising:
- a forming unit (2) comprising: a forming drum (6) comprising an outer circumferential surface, a plurality of forming pockets (10) arranged on the outer circumferential surface of said forming drum (6), each forming pocket (10) comprising a mould cavity; and
- a feeding unit (4) comprising:
∘ an apparatus for distributing fresh and/or recycled absorbent fibers (18) onto a substrate according to claim 1 to 4,
∘ at least one discharging conduits (22,22a, 22b) defined by a housing (26) with a internal wall fluidically connecting the outlets (27,100) of the defibrating station (12) and the forming unit (2) through which the fresh and/or recycled absorbent fibers (18) and a first airflow (24) flow.

6. Apparatus (1) suitable for forming moulded absorbent material deposit structures according to claim 5, wherein the apparatus further comprises an apparatus for distributing superabsorbent particles (34) comprising a discharge pipe (32) for conveying a stream (35) comprising superabsorbent particles (34) and a second airflow (62), said discharge pipe (32) comprising a conveying section (38) and an outlet nozzle, said outlet nozzle comprising a straight portion (42) and a deflecting portion (46) **characterized in that** the deflecting portion (46) comprises deflecting means (48) arranged in a way that only a portion (35a) of the stream (35) of superabsorbent particle (34) and second airflow (62) is deflected and the other portion (35b) of said stream is not deflected, wherein the discharge pipe (32) extends partially within the discharging conduit (22), the outlet nozzle being arranged within the discharging conduit (22) in a way that the discharge pipe (32) sprays superabsorbent particles (34) into the discharging conduit (22) and into the mould cavity of the forming pockets (10).

7. Apparatus (1) suitable for forming moulded absorbent material deposit structures according to claim 6, wherein the apparatus comprises two discharging conduit (22a,22b), each conduit (22a,22b) being fluidically connected to the defibrating station (12) and each conduit (22a,22b) comprising a discharge pipe (32) extending partially within its respective discharging conduit (22a,22b), the outlet nozzle of each pipe (32) being arranged within its respective discharging conduit (22a,22b) .

8. A method for forming moulded absorbent material deposit structures to be used as absorbent cores for absorbent articles using the apparatus according to claims 5 to 7, the method comprising:
a) a defibrating step wherein a continuous sheet of absorbent material (14) is defibrated by a defibrating machine (16) at a defibrating station (12), thereby obtaining a plurality of fresh absorbent fibers (18);
b) a conveying step where a stream of recycled absorbent fibers (18) is transported in the recycling duct (100);
c) a transport step wherein fresh and/or recycled absorbent fibers (18) are conveyed by a first airflow (24) flowing within the discharging conduit (22) or ducts (22a,22b) from the defibrating station (12) and recycling duct (100) to the forming pocket (10);
d) a feeding step wherein a specific amount of superabsorbent particles (34) in conjunction with the second airflow (62) is sprayed from the discharge pipe (32), into the discharging conduit (22) via the outlet nozzle;
e) an accumulation step wherein the fresh and/or recycled absorbent fibers (18) and the superabsorbent particles (34) accumulate within the mould cavity of the forming pocket (10) thereby forming moulded absorbent material deposit structures to be used as absorbent cores for absorbent articles.

## Patentansprüche

1. Vorrichtung zum Verteilen von absorbierenden Fasern (18) auf ein Substrat, wobei die Vorrichtung eine Defibrierstation (12) umfasst, umfassend:
a. eine Defibriermaschine (16), die zum Defibrieren einer Bahn aus Zellstoffmaterial (14) und zum Erzeugen von frischen absorbierenden Fasern (18) und einem ersten Luftstrom (24) geeignet ist, und
b. einen Recyclingkanal (100), der die Defibriermaschine (16) umgeht und recycelte absorbierende Fasern (18) aus einer Filtervorrichtung (210) fördert,
wobei die Defibriermaschine (16) und der Recyclingkanal (100) mit mindestens einem Kanal (22, 22a, 22b) fluidisch verbunden sind, wobei die Defibriermaschine (16) und der Recyclingkanal (100) mit mindestens zwei Kanälen (22, 22a, 22b) fluidisch verbunden sind, wobei der Recyclingkanal (100) mindestens eine Klappe (110) umfasst, um die recycelten absorbierenden Fasern (18) in einen, zwei oder mehrere der Kanäle (22, 22a, 22b) zu fördern.

2. Vorrichtung zum Verteilen von absorbierenden Fasern (18) auf ein Substrat nach Anspruch 1, wobei die Vorrichtung zwei Kanäle (22, 22a, 22b) umfasst und der Recyclingkanal (100) zwei Klappen (110) umfasst, um die recycelten absorbierenden Fasern (18) in einen oder zwei Kanäle (22, 22a, 22b) zu fördern.

3. Vorrichtung zum Verteilen von absorbierenden Fasern (18) auf ein Substrat nach Anspruch 1 oder 2, wobei die mindestens eine Klappe (110) aus einer Türklappe, einer Schmetterlingsklappe, einer Trommelklappe, einer Schiebetürklappe ausgewählt ist.

4. Vorrichtung zum Verteilen von absorbierenden Fasern (18) auf ein Substrat nach einem der vorhergehenden Ansprüche, wobei die Defibriermaschine (16) eine Hammermühle ist, die in einem Trommelgehäuse (23) angeordnet ist, wobei der Recyclingkanal (100) der Krümmung des Trommelgehäuses folgt.

5. Vorrichtung (1), die zum Bilden von geformten Ablagerungsstrukturen aus absorbierendem Material zur Verwendung als absorbierende Kerne für absorbierende Artikel geeignet ist, wobei die Vorrichtung (1) umfasst:
- eine Formungseinheit (2), umfassend: eine Formungstrommel (6), die eine äußere Umfangsfläche umfasst, eine Mehrzahl von Formungstaschen (10), die auf der äußeren Umfangsfläche der Formungstrommel (6) angeordnet sind, wobei jede Formungstasche (10) einen Formhohlraum umfasst; und
- eine Zuführeinheit (4), umfassend:
∘ eine Vorrichtung zum Verteilen von frischen und/oder recycelten absorbierenden Fasern (18) auf ein Substrat nach Anspruch 1 bis 4,
∘ mindestens eine Abgabeleitung (22, 22a, 22b), die durch ein Gehäuse (26) mit einer Innenwand definiert ist, die die Auslässe (27, 100) der Defibrierstation (12) und die Formungseinheit (2) fluidisch verbindet, durch die die frischen und/oder recycelten absorbierenden Fasern (18) und ein erster Luftstrom (24) strömen.

6. Vorrichtung (1), die zum Bilden von geformten Ablagerungsstrukturen aus absorbierendem Material nach Anspruch 5 geeignet ist, wobei die Vorrichtung ferner eine Vorrichtung zum Verteilen von superabsorbierenden Partikeln (34) umfasst, die ein Abgaberohr (32) zum Fördern eines Stroms (35) umfasst, der superabsorbierende Partikel (34) und einen zweiten Luftstrom (62) umfasst, wobei das Abgaberohr (32) einen Förderabschnitt (38) und eine Auslassdüse umfasst, wobei die Auslassdüse einen geraden Abschnitt (42) und einen Ablenkabschnitt (46) umfasst, **dadurch gekennzeichnet, dass** der Ablenkabschnitt (46) Ablenkmittel (48) umfasst, die so angeordnet sind, dass nur ein Teil (35a) des Stroms (35) aus superabsorbierenden Partikeln (34) und dem zweiten Luftstrom (62) abgelenkt wird und der andere Teil (35b) des Stroms nicht abgelenkt wird, wobei sich das Abgaberohr (32) teilweise innerhalb der Abgabeleitung (22) erstreckt, wobei die Auslassdüse innerhalb der Abgabeleitung (22) so angeordnet ist, dass das Abgaberohr (32) superabsorbierende Partikel (34) in die Abgabeleitung (22) und in den Formhohlraum der Formungstaschen (10) sprüht.

7. Vorrichtung (1), die zum Bilden von geformten Ablagerungsstrukturen aus absorbierendem Material nach Anspruch 6 geeignet ist, wobei die Vorrichtung zwei Abgabeleitungen (22a, 22b) umfasst, wobei jede Leitung (22a, 22b) mit der Defibrierstation (12) fluidisch verbunden ist und jede Leitung (22a, 22b) ein Abgaberohr (32) umfasst, das sich teilweise innerhalb seiner jeweiligen Abgabeleitung erstreckt, wobei die Auslassdüse jedes Rohrs (32) innerhalb ihrer jeweiligen Abgabeleitung angeordnet ist.

8. Verfahren zum Bilden von geformten Ablagerungsstrukturen aus absorbierendem Material zur Verwendung als absorbierende Kerne für absorbierende Artikel unter Verwendung der Vorrichtung nach den Ansprüchen 5 bis 7, wobei das Verfahren umfasst:
a) einen Defibrierschritt, bei dem eine kontinuierliche Bahn aus absorbierendem Material (14) durch eine Defibriermaschine (16) an einer Defibrierstation (12) defibriert wird, wodurch eine Mehrzahl von frischen absorbierenden Fasern (18) erhalten wird;
b) einen Förderschritt, bei dem ein Strom von recycelten absorbierenden Fasern (18) in dem Recyclingkanal (100) transportiert wird;
c) einen Transportschritt, bei dem frische und/oder recycelte absorbierende Fasern (18) durch einen ersten Luftstrom (24), der innerhalb der Abgabeleitung (22) oder der Kanäle (22a, 22b) strömt, von der Defibrierstation (12) und dem Recyclingkanal (100) zu der Formungstasche (10) gefördert werden;
d) einen Zuführschritt, bei dem eine bestimmte Menge an superabsorbierenden Partikeln (34) in Verbindung mit dem zweiten Luftstrom (62) aus dem Abgaberohr (32) über die Auslassdüse in die Abgabeleitung (22) gesprüht wird;
e) einen Akkumulationsschritt, bei dem sich die frischen und/oder recycelten absorbierenden Fasern (18) und die superabsorbierenden Partikel (34) innerhalb des Formhohlraums der Formungstasche (10) akkumulieren, wodurch geformte Ablagerungsstrukturen aus absorbierendem Material zur Verwendung als absorbierende Kerne für absorbierende Artikel gebildet werden.

## Revendications

1. Dispositif pour distribuer des fibres absorbantes (18) sur un substrat, l'dispositif comprenant une station de défibrage (12) comprenant :
a. une machine de défibrage (16) appropriée pour défibrer une feuille de matériau de pâte (14) et générer des fibres absorbantes fraîches (18) et un premier flux d'air (24), et
b. un conduit de recyclage (100) contournant la machine de défibrage (16) et acheminant des fibres absorbantes recyclées (18) à partir d'un dispositif de filtration (210),
dans lequel la machine de défibrage (16) et le conduit de recyclage (100) sont connectés de manière fluidique à au moins un conduit (22, 22a, 22b), dans lequel la machine de défibrage (16) et le conduit de recyclage (100) sont connectés de manière fluidique à au moins deux conduits (22, 22a, 22b), dans lequel le conduit de recyclage (100) comprend au moins un volet (110) pour acheminer les fibres absorbantes recyclées (18) dans un, deux ou plusieurs des conduits (22, 22a, 22b).

2. Dispositif pour distribuer des fibres absorbantes (18) sur un substrat selon la revendication 1, dans lequel l'dispositif comprend deux conduits (22, 22a, 22b) et le conduit de recyclage (100) comprend deux volets (110) pour acheminer les fibres absorbantes recyclées (18) dans un ou deux conduits (22, 22a, 22b).

3. Dispositif pour distribuer des fibres absorbantes (18) sur un substrat selon la revendication 1 ou 2, dans lequel ledit au moins un volet (110) est choisi parmi un volet de type porte, un volet papillon, un volet tambour, un volet de type porte coulissante.

4. Dispositif pour distribuer des fibres absorbantes (18) sur un substrat selon l'une quelconque des revendications précédentes, dans lequel la machine de défibrage (16) est un broyeur à marteaux agencé dans un boîtier de tambour (23), le conduit de recyclage (100) suivant la courbure dudit boîtier de tambour.

5. Dispositif (1) approprié pour former des structures de dépôt de matériau absorbant moulées destinées à être utilisées comme noyaux absorbants pour des articles absorbants, le dispositif (1) comprenant :
- une unité de formation (2) comprenant : un tambour de formation (6) comprenant une surface circonférentielle externe, une pluralité de poches de formation (10) agencées sur la surface circonférentielle externe dudit tambour de formation (6), chaque poche de formation (10) comprenant une cavité de moule ; et
- une unité d'alimentation (4) comprenant :
∘ un dispositif pour distribuer des fibres absorbantes (18) fraîches et/ou recyclées sur un substrat selon les revendications 1 à 4,
∘ au moins un conduit de décharge (22, 22a, 22b) défini par un boîtier (26) avec une paroi interne connectant de manière fluidique les sorties (27, 100) de la station de défibrage (12) et de l'unité de formation (2) à travers lequel s'écoulent les fibres absorbantes (18) fraîches et/ou recyclées et un premier flux d'air (24).

6. Dispositif (1) approprié pour former des structures de dépôt de matériau absorbant moulées selon la revendication 5, dans lequel l'dispositif comprend en outre un dispositif pour distribuer des particules superabsorbantes (34) comprenant un tuyau de décharge (32) pour acheminer un flux (35) comprenant des particules superabsorbantes (34) et un second flux d'air (62), ledit tuyau de décharge (32) comprenant une section d'acheminement (38) et une buse de sortie, ladite buse de sortie comprenant une portion droite (42) et une portion de déviation (46), **caractérisé en ce que** la portion de déviation (46) comprend des moyens de déviation (48) agencés de telle manière que seule une portion (35a) du flux (35) de particules superabsorbantes (34) et du second flux d'air (62) est déviée et l'autre portion (35b) dudit flux n'est pas déviée, dans lequel le tuyau de décharge (32) s'étend partiellement à l'intérieur du conduit de décharge (22), la buse de sortie étant agencée à l'intérieur du conduit de décharge (22) de telle manière que le tuyau de décharge (32) pulvérise des particules superabsorbantes (34) dans le conduit de décharge (22) et dans la cavité de moule des poches de formation (10).

7. Dispositif (1) approprié pour former des structures de dépôt de matériau absorbant moulées selon la revendication 6, dans lequel l'dispositif comprend deux conduits de décharge (22a, 22b), chaque conduit (22a, 22b) étant connecté de manière fluidique à la station de défibrage (12) et chaque conduit (22a, 22b) comprenant un tuyau de décharge (32) s'étendant partiellement à l'intérieur de son conduit de décharge respectif, la buse de sortie de chaque tuyau (32) étant agencée à l'intérieur de son conduit de décharge (22a, 22b) respectif.

8. Procédé pour former des structures de dépôt de matériau absorbant moulées destinées à être utilisées comme noyaux absorbants pour des articles absorbants en utilisant l'dispositif selon les revendications 5 à 7, le procédé comprenant :
a) une étape de défibrage dans laquelle une feuille continue de matériau absorbant (14) est défibrée par une machine de défibrage (16) au niveau d'une station de défibrage (12), obtenant ainsi une pluralité de fibres absorbantes fraîches (18) ;
b) une étape d'acheminement dans laquelle un flux de fibres absorbantes recyclées (18) est transporté dans le conduit de recyclage (100) ;
c) une étape de transport dans laquelle des fibres absorbantes (18) fraîches et/ou recyclées sont acheminées par un premier flux d'air (24) s'écoulant à l'intérieur du conduit de décharge (22) ou des conduits (22a, 22b) depuis la station de défibrage (12) et le conduit de recyclage (100) vers la poche de formation (10) ;
d) une étape d'alimentation dans laquelle une quantité spécifique de particules superabsorbantes (34) conjointement avec le second flux d'air (62) est pulvérisée depuis le tuyau de décharge (32), à l'intérieur du conduit de décharge (22) via la buse de sortie ;
e) une étape d'accumulation dans laquelle les fibres absorbantes (18) fraîches et/ou recyclées et les particules superabsorbantes (34) s'accumulent à l'intérieur de la cavité de moule de la poche de formation (10) formant ainsi des structures de dépôt de matériau absorbant moulées destinées à être utilisées comme noyaux absorbants pour des articles absorbants.
